# EUROPEAN PATENT SPECIFICATION

(11) **EP 4 582 024 B1**
(45) Date of publication and mention of the grant of the patent: **29.07.2026**
(21) Application number: 23859937.7
(22) Date of filing: 31.07.2023
(51) Int. Cl.: A61B 6/00

(54) **INFORMATION PROCESSING DEVICE, INFORMATION PROCESSING METHOD, AND PROGRAM**
INFORMATIONSVERARBEITUNGSVORRICHTUNG, INFORMATIONSVERARBEITUNGSVERFAHREN UND PROGRAMM
DISPOSITIF DE TRAITEMENT D'INFORMATIONS, PROCÉDÉ DE TRAITEMENT D'INFORMATIONS ET PROGRAMME

(30) Priority: 02.09.2022 JP 2022140181
(43) Date of publication of application: 09.07.2025
(73) Proprietor: FUJIFILM Corporation, Tokyo 106-8620 (JP)
(72) Inventor: MACHII, Yusuke, Ashigarakami-gun, Kanagawa 258-8538 (JP)
(74) Representative: Dehns Germany Partnerschaft mbB
(86) International application number: PCT/JP2023/028031
(87) International publication number: WO 2024/048168

(56) References cited:
- WO-A1-2019/017442
- WO-A1-2022/065318
- CN-A- 113 288 186
- JP-A- 2002 521 896
- JP-A- 2020 010 805
- JP-A- 2020 116 283
- JP-A- H04 156 789

## Description

### BACKGROUND OF THE INVENTION

### 1. Field of the Invention

The present disclosure relates to an information processing apparatus, an information processing method, and a program.

### 2. Description of the Related Art

Contrast-enhanced mammography, which acquires a low-energy image and a high-energy image by performing imaging by irradiating a breast in which a contrast agent is injected with radiation having different energies and generates a difference image representing a difference between the low-energy image and the high-energy image to generate an image in which a lesion or the like is contrast-enhanced, is known. In recent years, since the contrast-enhanced mammography has been included in a comprehensive guideline for breast cancer image diagnosis called a breast imaging reporting and data system (BI-RADS), there is a high possibility that the contrast-enhanced mammography will be widely used as a standard diagnosis method.

However, it is difficult to perform the interpretation of the image obtained by the contrast-enhanced mammography. One of the reasons for the difficulty is an effect of background mammary gland parenchymal enhancement (BPE) due to the contrast agent. The BPE represents a level of enhancement of a normal structure of a mammary gland via the contrast agent, and the visibility of the enhanced lesion greatly varies depending on the level of the BPE. As described above, since the difficulty of the interpretation is high in the contrast-enhanced mammography, it is desired to support even a doctor who is not accustomed to the interpretation so that standard interpretation can be performed.

As a technology related to supporting the interpretation of the image in mammography, for example, in Richa Agarwal, et al., 'Deep learning for mass detection in Full Field Digital Mammograms', [online]; Computers in Biology and Medicine 121 (2020) 103774, [retrieved on 2022-08-16]. Retrieved from the Internet: <URL: https://www.sciencedirect.com/science/article/pii/S001048252030144X>., it is proposed to detect a lesion region including a lesion such as breast cancer by using a Faster Region-based Convolutional Neural Network (R-CNN). WO 2022/065318 A1 discloses an information processing apparatus for analysing dual-energy radiation images of a subject injected with a contrast agent, including the generation of a difference image from low-energy and high-energy images and the detection of lesions using machine learning techniques.

### SUMMARY OF THE INVENTION

The invention is defined by the appended claims. In BI-RADS, in a case in which a finding is found by interpretation, the doctor needs to clearly write in the report whether the finding is found only in the low-energy image, only in the difference image, or in both the low-energy image and the difference image.

Richa Agarwal, et al., 'Deep learning for mass detection in Full Field Digital Mammograms', [online]; Computers in Biology and Medicine 121 (2020) 103774, [retrieved on 2022-08-16]. Retrieved from the Internet: <URL: https://www.sciencedirect.com/science/article/pii/S001048252030144X>. describes that a detection position of the lesion region via lesion region detection is shown in an image, but the technology described in Richa Agarwal, et al., 'Deep learning for mass detection in Full Field Digital Mammograms', [online]; Computers in Biology and Medicine 121 (2020) 103774, [retrieved on 2022-08-16]. Retrieved from the Internet: <URL: https://www.sciencedirect.com/science/article/pii/S001048252030144X>. does not perform contrast enhancement via the energy subtraction as described above. Therefore, in the related art, it is not possible to discriminate whether the lesion region detected by the lesion region detection is detected from the low-energy image or the difference image, so that it is not possible to sufficiently support the interpretation of the image.

An object of the present disclosed technology is to provide an information processing apparatus, an information processing method, and a program capable of improving support for interpretation of an image generated by contrast-enhanced imaging.

In order to achieve the above-described object, the present invention provides an information processing apparatus as defined by claim 1.

It is preferable that the processor is configured to: display the comparison determination result on a display along with the low-energy image and the difference image.

It is preferable that the processor is configured to: selectively display the low-energy image and the difference image on a display along with the comparison determination result in response to an operation signal transmitted from an operation unit.

It is preferable that the processor is configured to: display a finding on a display along with the comparison determination result.

It is preferable that the subject is left and right breasts, the low-energy image includes a first low-energy image and a second low-energy image that are captured by irradiating each of the left and right breasts with radiation having the first energy, the high-energy image includes a first high-energy image and a second high-energy image that are captured by irradiating each of the left and right breasts with radiation having the second energy, and the difference image includes a first difference image representing a difference between the first low-energy image and the first high-energy image and a second difference image representing a difference between the second low-energy image and the second high-energy image.

It is preferable that the processor is configured to: selectively display the first low-energy image, the second low-energy image, the first difference image, and the second difference image on a display along with the comparison determination result in response to an operation signal transmitted from an operation unit.

The present invention also provides an information processing method as defined by claim 7.

The present invention also provides a program as defined by claim 8.

According to the present disclosed technology, it is possible to provide the information processing apparatus, the information processing method, and the program capable of improving the support for the interpretation of the image generated by the contrast-enhanced imaging.

### BRIEF DESCRIPTION OF THE DRAWINGS

Fig. 1 is a diagram showing an example of an overall configuration of a radiation image capturing system according to a first embodiment.
Fig. 2 is a block diagram showing an example of a configuration of an information processing apparatus.
Fig. 3 is a block diagram showing an example of functions implemented by a control unit of the information processing apparatus.
Fig. 4 is a flowchart schematically showing a flow of contrast-enhanced imaging processing.
Fig. 5 is a flowchart schematically showing a flow of detection processing.
Fig. 6 is a diagram schematically showing lesion region detection processing via a lesion region detection processing unit.
Fig. 7 is a diagram schematically showing detection position comparison determination processing via a detection position comparison determination processing unit.
Fig. 8 is a diagram schematically showing display processing via a display control unit.
Fig. 9 is a diagram schematically showing lesion region detection processing according to a first modification example.
Fig. 10 is a diagram schematically showing display processing according to a second modification example.
Fig. 11 is a diagram schematically showing display processing according to a third modification example.
Fig. 12 is a diagram schematically showing lesion region detection processing according to a fourth modification example.
Fig. 13 is a diagram schematically showing display processing according to the fourth modification example.

### DESCRIPTION OF THE PREFERRED EMBODIMENTS

Hereinafter, an embodiment of the present disclosure will be described in detail with reference to the accompanying drawings.

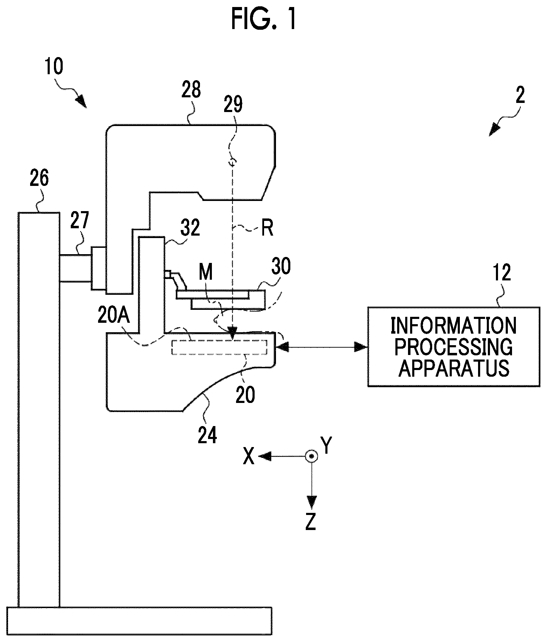

Fig. 1 shows an example of an overall configuration of a radiation image capturing system 2. The radiation image capturing system 2 comprises a mammography apparatus 10 and an information processing apparatus 12. The information processing apparatus 12 is connected to a radiology information system (RIS), a picture archiving and communication system (PACS), and the like (none of which is shown) via a network or the like.

Fig. 1 shows an example of an appearance of the mammography apparatus 10. It should be noted that Fig. 1 shows an example of the appearance in a case in which the mammography apparatus 10 is seen from a left side of a person under an examination.

The mammography apparatus 10 is a radiography apparatus that operates under the control of the information processing apparatus 12 and that irradiates a breast M of a person under an examination, as a subject, with radiation R (for example, X-rays) from a radiation source 29 to capture a radiation image of the breast M.

As shown in Fig. 1, the mammography apparatus 10 comprises an imaging table 24, a base 26, an arm part 28, and a compression unit 32. A radiation detector 20 is disposed inside the imaging table 24. As shown in Fig. 1, in a case in which imaging is performed, in the mammography apparatus 10, the breast M of the person under an examination is positioned on the imaging table 24 by a user, such as a radiologist.

The radiation detector 20 detects the radiation R passing through the breast M as the subject. Specifically, the radiation detector 20 detects the radiation R passing through the breast M of the person under an examination, entering into the imaging table 24, and reaching a detection surface 20A of the radiation detector 20, and generates a radiation image based on the detected radiation R. The radiation detector 20 outputs image data representing the generated radiation image. Hereinafter, the series of operations of irradiating the breast with the radiation R from the radiation source 29 to generate the radiation image via the radiation detector 20 may be referred to as "imaging". The radiation detector 20 may be an indirect conversion type radiation detector that converts the radiation R into light beams and converts the converted light beams into charges, or may be a direct conversion type radiation detector that directly converts the radiation R into charges.

Hereinafter, two directions orthogonal to each other and parallel to the detection surface 20A will be referred to as an X direction and a Y direction. In addition, a direction orthogonal to the X direction and the Y direction will be referred to as a Z direction.

A compression plate 30 that is used for compressing the breast M in a case of performing the imaging is attached to the compression unit 32. The compression plate 30 is moved in a direction approaching or in a direction spaced away from the imaging table 24 by a compression plate drive unit (not shown) provided in the compression unit 32. The compression plate 30 is moved in a direction approaching the imaging table 24 to compress the breast M with the imaging table 24.

The arm part 28 can be rotated with respect to the base 26 by a shaft part 27. The shaft part 27 is fixed to the base 26, and the shaft part 27 and the arm part 28 are rotated integrally. Gears are provided in each of the shaft part 27 and the compression unit 32 of the imaging table 24, and the gears are switched between an engaged state and a non-engaged state, so that a state in which the compression unit 32 of the imaging table 24 and the shaft part 27 are connected to each other and are rotated integrally and a state in which the shaft part 27 is separated from the imaging table 24 and idles can be switched. The elements for switching between transmission and non-transmission of power of the shaft part 27 are not limited to the gears, and various mechanical elements can be used. The arm part 28 and the imaging table 24 can be separately rotated relative to the base 26 with the shaft part 27 as a rotation axis.

The mammography apparatus 10 can perform the imaging on each of the left and right breasts M from a plurality of directions by rotating the arm part 28. For example, it is possible to perform cranio-caudal (CC) imaging and medio-lateral oblique (MLO) imaging.

The radiation image capturing system 2 can perform "contrast-enhanced imaging" in which the imaging is performed in a state in which a contrast agent is injected in the breast M. Specifically, the radiation image capturing system 2 has a contrast enhanced digital mammography (CEDM) function of performing contrast enhancement via energy subtraction.

In the contrast-enhanced imaging, a low-energy image and a high-energy image are acquired by performing the imaging by irradiating the breast M, in which the contrast agent is injected, with the radiation R having different energies. In the present disclosure, a radiation image captured by the radiation R having a first energy will be referred to as a "low-energy image", and a radiation image captured by the radiation R having a second energy higher than the first energy will be referred to as a "high-energy image". Hereinafter, in a case in which the low-energy image and the high-energy image are not distinguished from each other, the low-energy image and the high-energy image will be simply referred to as a radiation image.

In the contrast-enhanced imaging, for example, an iodine contrast agent having a k absorption edge of 32 keV is used as the contrast agent. In the contrast-enhanced imaging in a case in which the iodine contrast agent is used, the first energy need only be set to be lower than the k absorption edge, and the second energy need only be set to be higher than the k absorption edge.

The contrast agent and the body tissue such as the mammary gland are different in absorption characteristics of the radiation R. Therefore, the high-energy image clearly shows the contrast agent in addition to the body tissue such as the mammary gland and the fat. On the other hand, in the low-energy image, the body tissue is clearly shown, but the contrast agent is hardly shown. Therefore, by taking a difference between the low-energy image and the high-energy image, it is possible to generate a difference image in which the mammary gland structure is erased and a lesion or the like stained with the contrast agent is enhanced. The lesion consists of, for example, new cells and is easily stained with the contrast agent.

The mammography apparatus 10 and the information processing apparatus 12 are connected by wired communication or wireless communication. The radiation image generated by the radiation detector 20 in the mammography apparatus 10 is output to the information processing apparatus 12 by wired communication or wireless communication via a communication interface (I/F) (not shown).

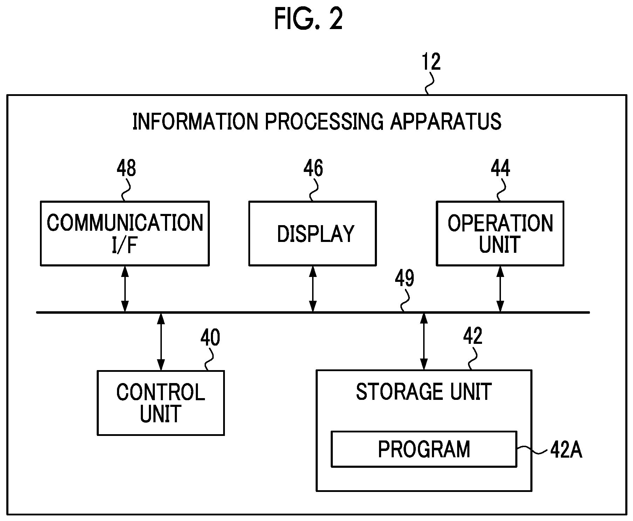

Fig. 2 shows an example of the configuration of the information processing apparatus 12. The information processing apparatus 12 comprises a control unit 40, a storage unit 42, an operation unit 44, a display 46, and a communication I/F 48. The control unit 40, the storage unit 42, the operation unit 44, the display 46, and the communication I/F 48 are connected to each other via a bus 49 such that various kinds of information can be exchanged.

The control unit 40 controls an overall operation of the radiation image capturing system 2. The control unit 40 is configured by, for example, a computer comprising a central processing unit (CPU), a read only memory (ROM), and a random access memory (RAM).

The storage unit 42 stores information related to radiography, the radiation image acquired from the mammography apparatus 10, and the like. In addition, the storage unit 42 stores a program 42A for the control unit 40 to perform various kinds of information processing described later and data for constructing various kinds of machine learned models described later. The storage unit 42 is, for example, a nonvolatile storage device such as a hard disk drive (HDD) or a solid state drive (SSD).

The operation unit 44 includes input devices such as various buttons, switches, a touch panel, a touch pen, and a mouse, which are operated by the user. The display 46 displays information related to imaging, a radiation image obtained by imaging, a comparison determination result obtained by detection position comparison determination processing described later, and the like.

The communication I/F 48 performs communication of various kinds of data, such as information related to the radiography and the radiation image, with the mammography apparatus 10, the RIS, the PACS, and the like via wired communication or wireless communication.

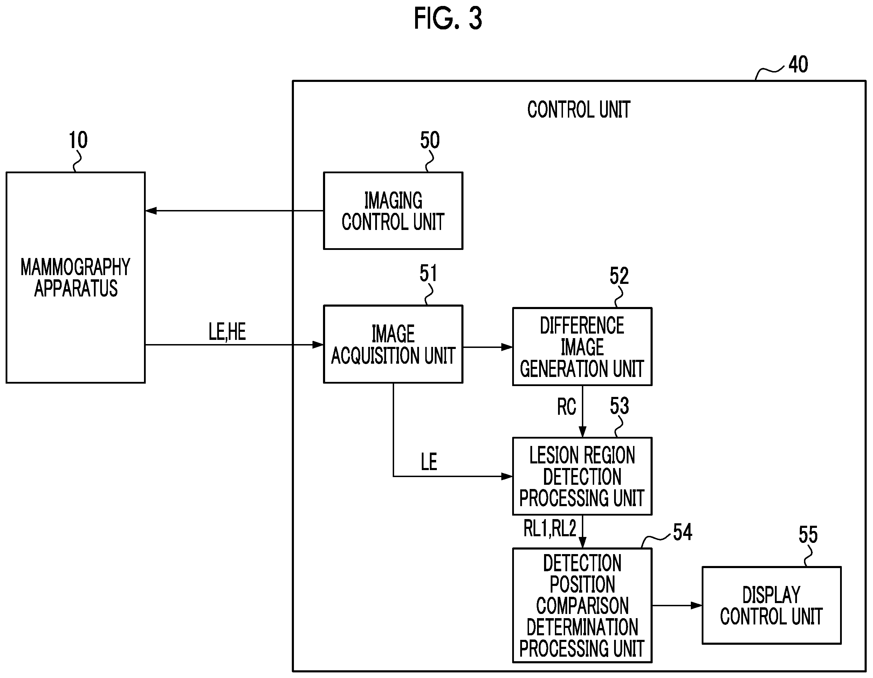

Fig. 3 shows an example of functions implemented by the control unit 40 of the information processing apparatus 12. The control unit 40 implements various functions by executing the processing based on the program 42A stored in the storage unit 42. The control unit 40 functions as an imaging control unit 50, an image acquisition unit 51, a difference image generation unit 52, a lesion region detection processing unit 53, a detection position comparison determination processing unit 54, and a display control unit 55.

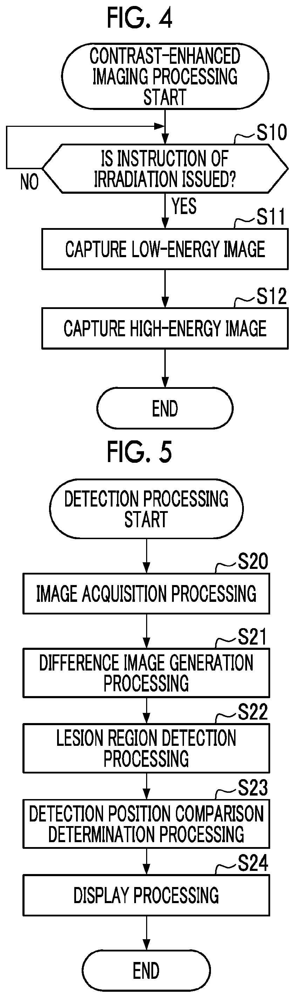

Fig. 4 schematically shows a flow of contrast-enhanced imaging processing. Processing via the imaging control unit 50 will be described with reference to Fig. 4.

First, before the imaging via the mammography apparatus 10 is started, the user, such as the radiologist, injects the contrast agent into the breast M of the person under an examination, positions the breast M in which the contrast agent is injected on the imaging table 24, and compresses the breast M with the compression plate 30.

In step S10, the imaging control unit 50 determines whether or not an instruction of the irradiation with the radiation R is received. In a case in which the instruction of the irradiation is received, the imaging control unit 50 outputs, in step S11, an instruction of the irradiation with the radiation R having the first energy to the mammography apparatus 10. In the mammography apparatus 10, a low-energy image LE is captured by emitting the first energy radiation R toward the breast M.

In next step S12, the imaging control unit 50 outputs an instruction of the irradiation with the radiation R having the second energy to the mammography apparatus 10. In the mammography apparatus 10, a high-energy image HE is captured by emitting the radiation R having the second energy toward the breast M. It should be noted that the high-energy image HE may be captured earlier than the low-energy image LE.

In a case in which the capturing of the low-energy image LE and the high-energy image HE of the breast M ends, the user releases the compression of the breast M for which the imaging ends.

Fig. 5 schematically shows a flow of detection processing. Processing via the imaging control unit 50, the image acquisition unit 51, the difference image generation unit 52, the lesion region detection processing unit 53, the detection position comparison determination processing unit 54, and the display control unit 55 will be described with reference to Fig. 5.

In step S20, the image acquisition unit 51 acquires the low-energy image LE and the high-energy image HE captured by the above-described contrast-enhanced imaging processing.

In next step S21, the difference image generation unit 52 generates a difference image RC representing a difference between the low-energy image LE and the high-energy image HE. For example, the difference image generation unit 52 generates the difference image RC by subtracting an image obtained by multiplying the low-energy image LE by a first weight coefficient from an image obtained by multiplying the high-energy image HE by a second weight coefficient for each corresponding pixel.

In next step S22, the lesion region detection processing unit 53 performs lesion region detection processing of detecting a region (hereinafter, referred to as a lesion region) including a lesion from each of the low-energy image LE and the difference image RC.

In next step S23, the detection position comparison determination processing unit 54 performs detection position comparison determination processing of performing a comparison determination of a position of the lesion region detected from the low-energy image LE and a position of the lesion region detected from the difference image RC. The comparison determination result obtained by the detection position comparison determination processing includes information indicating whether each of the lesion regions detected by the lesion region detection processing is the region detected based on the low-energy image LE or the difference image RC.

In next step S24, the display control unit 55 performs display processing of displaying the comparison determination result of the detection position comparison determination processing on the display 46, along with the low-energy image LE and the difference image RC. It should be noted that the display control unit 55 is an example of a "comparison determination result output unit" according to the present disclosed technology.

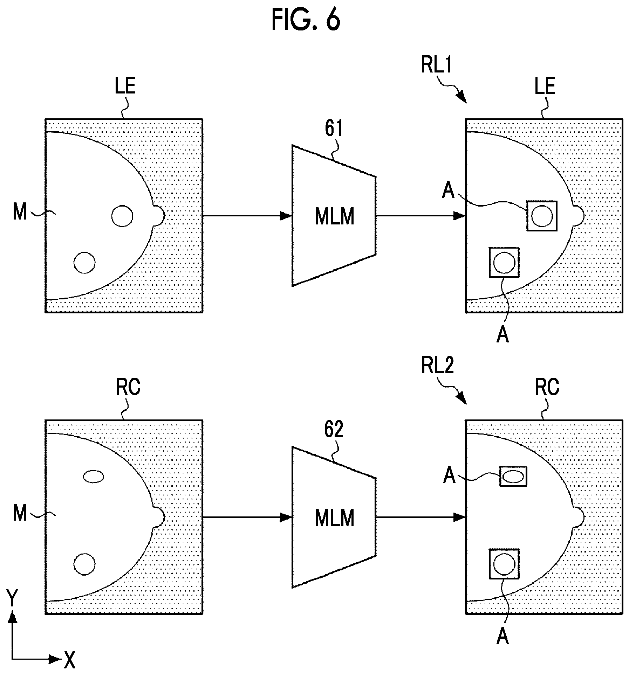

Fig. 6 schematically shows the lesion region detection processing via the lesion region detection processing unit 53. The lesion region detection processing unit 53 detects the lesion region from the low-energy image LE by inputting the low-energy image LE to a first machine learned model (MLM) 61 that functions as a first lesion region detection unit. In addition, the lesion region detection processing unit 53 detects the lesion region from the difference image RC by inputting the difference image RC to a second MLM 62 that functions as a second lesion region detection unit.

Each of the first MLM 61 and the second MLM 62 is configured by a convolutional neural network (CNN). For example, the first MLM 61 and the second MLM 62 are configured by an R-CNN or the like that detects an object from an image.

For example, each of the first MLM 61 and the second MLM 62 performs three kinds of processing of region proposal processing, feature value extraction processing, and class classification processing, to detect a lesion region A from the low-energy image LE and the difference image RC. The first MLM 61 outputs positional information of the lesion region A detected based on the low-energy image LE, as a first lesion region detection result RL1. The second MLM 62 outputs positional information of the lesion region A detected based on the difference image RC, as a second lesion region detection result RL2.

The first MLM 61 is, for example, a machine learned model for the low-energy image LE generated by training a machine learning model through machine learning using, as training data, the low-energy image LE and the ground-truth data representing the position of the true lesion region. The second MLM 62 is, for example, a machine learned model for the difference image RC generated by training a machine learning model through machine learning using, as training data, the difference image RC and the ground-truth data representing the position of the true lesion region.

It should be noted that the first MLM 61 and the second MLM 62 may be configured by, for example, a U-net or the like, which is one kind of a CNN, to detect the lesion region A via the segmentation.

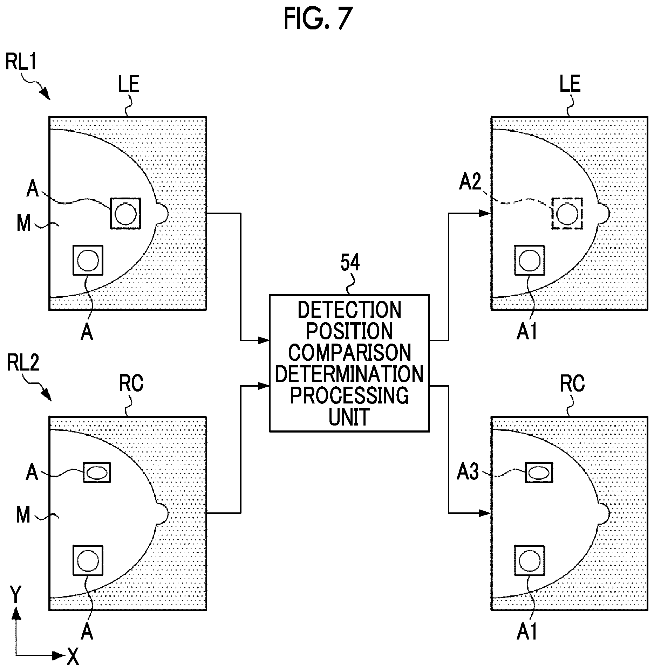

Fig. 7 schematically shows detection position comparison determination processing via the detection position comparison determination processing unit 54. The detection position comparison determination processing unit 54 compares the position of the lesion region A detected by the first MLM 61 with the position of the lesion region A detected by the second MLM 62, based on the first lesion region detection result RL1 and the second lesion region detection result RL2. Then, the detection position comparison determination processing unit 54 determines whether the lesion region A is detected by only one of the first MLM 61 or the second MLM 62 or detected by both the first MLM 61 and the second MLM 62.

Specifically, the detection position comparison determination processing unit 54 classifies each of the lesion regions A into a first lesion region A1, a second lesion region A2, and a third lesion region A3, and displays a classification result on the display 46 as the comparison determination result. The first lesion region A1 is the lesion region A detected by both the first MLM 61 and the second MLM 62. The second lesion region A2 is the lesion region A detected only by the first MLM 61 out of the first MLM 61 and the second MLM 62. The third lesion region A3 is the lesion region A detected only by the second MLM 62 out of the first MLM 61 and the second MLM 62. That is, the first lesion region A1 indicates a lesion seen in both the low-energy image LE and the difference image RC. The second lesion region A2 indicates a lesion seen only in the low-energy image LE. The third lesion region A3 indicates a lesion seen only in the difference image RC.

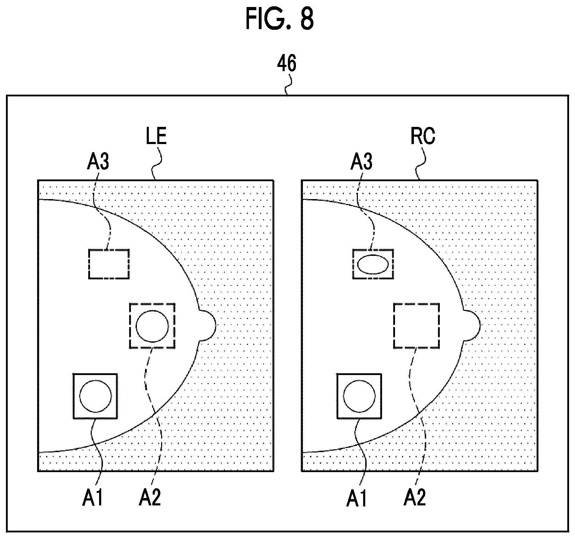

Fig. 8 schematically shows the display processing via the display control unit 55. The display control unit 55 displays the low-energy image LE and the difference image RC side by side on the display 46 and displays the comparison determination result obtained by the detection position comparison determination processing. The display control unit 55 displays the first lesion region A1, the second lesion region A2, and the third lesion region A3 on the low-energy image LE and the difference image RC in a distinguishable manner. For example, the display control unit 55 makes the first lesion region A1, the second lesion region A2, and the third lesion region A3 distinguishable by changing the colors, line types, and the like of rectangular frames indicating the first lesion region A1, the second lesion region A2, and the third lesion region A3, respectively.

As described above, in the present embodiment, the comparison determination of the position of the lesion region A detected from the low-energy image LE and the position of the lesion region A detected from the difference image RC is performed, and the comparison determination result is displayed. As a result, the user can easily discriminate whether the lesion region A is detected in the low-energy image LE or the difference image RC, so that the support for the interpretation of the image generated by the contrast-enhanced imaging is improved.

Hereinafter, various modification examples of the above-described embodiment will be described.

### [First Modification Example]

In the above-described embodiment, the lesion region detection processing unit 53 inputs the difference image RC to the second MLM 62, but, in the first modification example, the lesion region detection processing unit 53 inputs the low-energy image LE to the second MLM 62 in addition to the difference image RC.

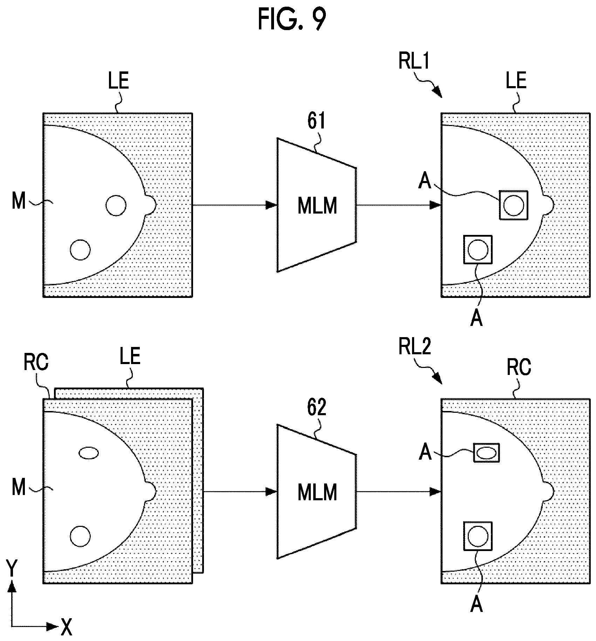

Fig. 9 schematically shows lesion region detection processing according to the first modification example. In the present modification example, the lesion region detection processing unit 53 inputs the low-energy image LE to the first MLM 61, and combines the difference image RC and the low-energy image LE to input the combined image to the second MLM 62. For example, the lesion region detection processing unit 53 combines the difference image RC and the low-energy image LE in the channel direction, and inputs the combined image to the second MLM 62. The channel direction is a direction in which a plurality of channels generated by the second MLM 62 via the convolution processing are combined. In the present modification example, the lesion region A is detected based on the difference image RC and the low-energy image LE. Other processing is the same as the processing according to the above-described embodiment.

According to the present modification example, the difference image RC and the low-energy image LE are input to the second MLM 62, so that the lesion region A can be detected with higher accuracy from the difference image RC.

### [Second Modification Example]

In the above-described embodiment, the display control unit 55 displays the low-energy image LE and the difference image RC side by side, but, in a second modification example, the display control unit 55 selectively displays the low-energy image LE and the difference image RC in response to an operation signal transmitted from the operation unit 44.

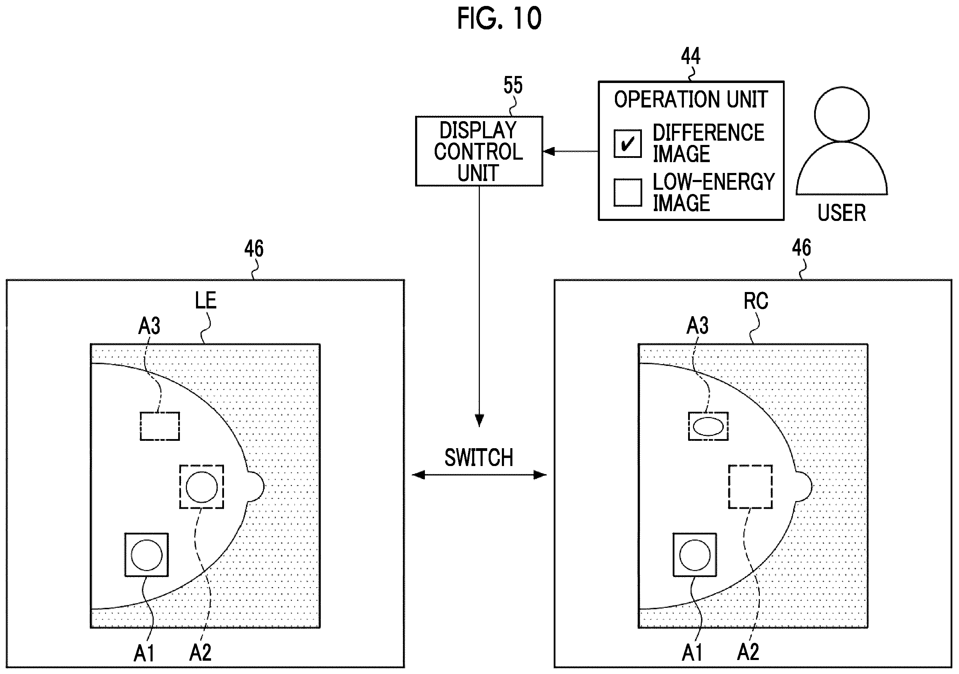

Fig. 10 schematically shows display processing according to the second modification example. In the present modification example, the display control unit 55 switches the images to be displayed on the display 46 based on the operation signal transmitted from the operation unit 44. The user can select whether to display the low-energy image LE or the difference image RC on the display 46 by operating the operation unit 44. The display control unit 55 displays the image selected by the user using the operation unit 44 out of the low-energy image LE and the difference image RC on the display 46.

### [Third Modification Example]

In the above-described embodiment, in the present embodiment, the lesion region A is displayed on the low-energy image LE and the difference image RC, but, in the third modification example, the display control unit 55 displays a finding in addition to the lesion region A. Examples of the finding include determinations related to a tumor, calcification, a locally asymmetric shadow (FAD), a construction disorder, and the like.

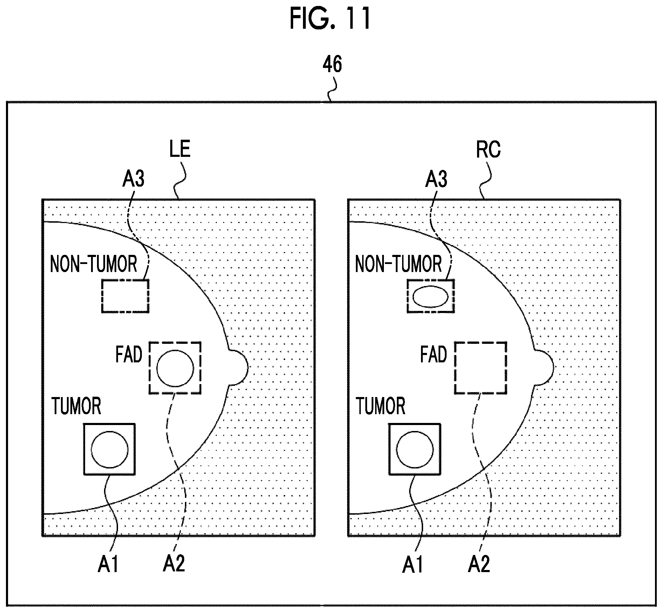

Fig. 11 schematically shows display processing according to the third modification example. In the present modification example, the display control unit 55 displays the low-energy image LE and the difference image RC on the display 46, and displays the finding in text in the vicinity of each of the first lesion region A1, the second lesion region A2, and the third lesion region A3.

In the present modification example, it is preferable that each of the first MLM 61 and the second MLM 62 is configured to detect the lesion region A and determine the finding for the lesion included in the lesion region A. In this case, the display control unit 55 displays the findings determined by the first MLM 61 and the second MLM 62, on the display 46.

It should be noted that the first MLM 61 and the second MLM 62 may include separate machine learned models for each finding. For example, the first MLM 61 and the second MLM 62 may include a machine learned model for detecting calcification, a machine learned model for detecting a tumor, and the like.

In addition, the display control unit 55 may display the finding for the lesion included in the second lesion region A2 detected only in the low-energy image LE and the lesion included in the first lesion region A1 detected in both the low-energy image LE and the difference image RC. For the lesion included in the first lesion region A1 detected in both the low-energy image LE and the difference image RC, a determination result of whether or not the lesion is a tumor may be displayed.

In addition, the display control unit 55 may limit the type of the finding to be displayed on the display 46 in response to the operation signal transmitted from the operation unit 44. That is, it is possible for the user to display only a desired finding on the display 46 via the operation unit 44.

### [Fourth Modification Example]

The fourth modification example shows an example in which the lesion region detection processing is performed on the left and right breasts M. In the present modification example, the image acquisition unit 51 acquires the low-energy image LE and the high-energy image HE captured by the contrast-enhanced imaging processing for each of the left and right breasts M. Hereinafter, the low-energy image LE and the high-energy image HE for the left breast M will be referred to as a "first low-energy image LE1" and a "first high-energy image HE1", respectively. In addition, the low-energy image LE and the high-energy image HE for the right breast M will be referred to as a "second low-energy image LE2" and a "second high-energy image HE2", respectively.

In the present modification example, the difference image generation unit 52 generates the difference image RC representing the difference between the low-energy image LE and the high-energy image HE for each of the left and right breasts M. Hereinafter, the difference image RC representing the difference between the first low-energy image LE1 and the first high-energy image HE1 will be referred to as a "first difference image RC1", and the difference image RC representing the difference between the second low-energy image LE2 and the second high-energy image HE2 will be referred to as a "second difference image RC2".

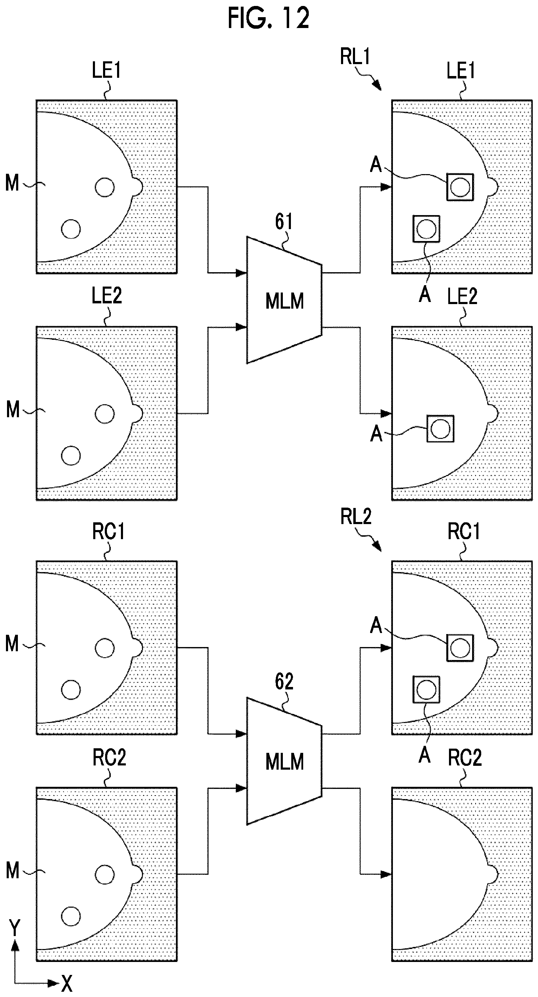

Fig. 12 schematically shows lesion region detection processing according to the fourth modification example. In the present modification example, the lesion region detection processing unit 53 detects the lesion regions A from the first low-energy image LE1 and the second low-energy image LE2 by inputting each of the first low-energy image LE1 and the second low-energy image LE2 to the first MLM 61. In addition, the lesion region detection processing unit 53 detects the lesion regions A from the first difference image RC1 and the second difference image RC2 by inputting each of the first difference image RC1 and the second difference image RC2 to the second MLM 62.

It should be noted that, in order to detect the lesion region from the first low-energy image LE1, the second low-energy image LE2 may be used in addition to the first low-energy image LE1. In addition, in order to detect the lesion region from the second low-energy image LE2, the first low-energy image LE1 may be used in addition to the second low-energy image LE2. In this case, the lesion region detection processing unit 53 combines the first low-energy image LE1 and the second low-energy image LE2, and inputs the combined image to the first MLM 61. As a result, the lesion region can be detected in consideration of the symmetry of the left and right breasts M. Similarly, the lesion region detection processing unit 53 may combine the first difference image RC1 and the second difference image RC2, and input the combined image to the second MLM 62.

In the present modification example, the detection position comparison determination processing unit 54 compares the position of the lesion region A detected by the first MLM 61 with the position of the lesion region A detected by the second MLM 62, for the first low-energy image LE1 and the first difference image RC1. In addition, the detection position comparison determination processing unit 54 compares the position of the lesion region A detected by the first MLM 61 with the position of the lesion region A detected by the second MLM 62, for the second low-energy image LE2 and the second difference image RC2. The detection position comparison determination processing unit 54 determines whether the lesion region Ais detected by only one of the first MLM 61 or the second MLM 62 or detected by both the first MLM 61 and the second MLM 62.

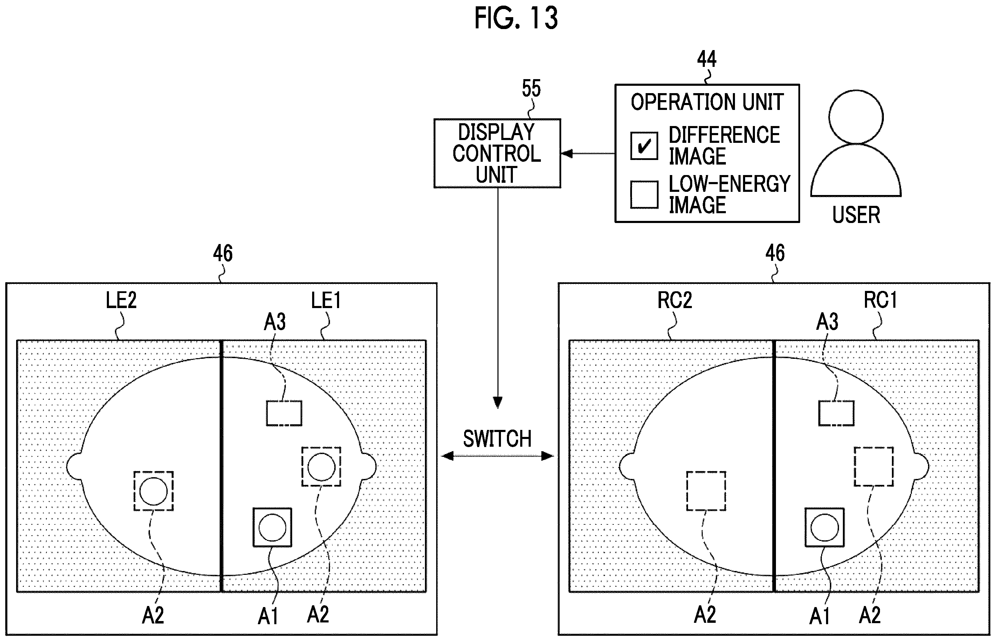

Fig. 13 schematically shows display processing according to the fourth modification example. In the present modification example, the display control unit 55 selectively displays the first low-energy image LE1 and the second low-energy image LE2, and the first difference image RC1 and the second difference image RC2 in response to the operation signal transmitted from the operation unit 44. The display control unit 55 switches the images to be displayed on the display 46 based on the operation signal transmitted from the operation unit 44, as in the second modification example. The user can select whether to display the first low-energy image LE1 and the second low-energy image LE2 or the first difference image RC1 and the second difference image RC2 on the display 46 by operating the operation unit 44.

### [Other Modification Examples]

In the above-described embodiment and respective modification examples, the display control unit 55 is the comparison determination result output unit, but the comparison determination result output unit is not limited to the display control unit 55, and may be any unit as long as the comparison determination result is output to the outside. That is, the control unit 40 may output the comparison determination result obtained by the detection position comparison determination processing to the external device via the communication I/F 48 or the like. For example, the control unit 40 outputs the image to the external device along with the comparison determination result. Here, the image output along with the comparison determination result may be one or a plurality of images.

As an example, the control unit 40 outputs the frames or the like indicating the first lesion region A1, the second lesion region A2, and the third lesion region A3, which indicate the comparison determination result, in a form of being superimposed on the low-energy image LE.

The image on which the comparison determination result is superimposed is not limited to the low-energy image LE, and may be the difference image RC or the high-energy image HE, or may be another image. For example, the image on which the comparison determination result is superimposed may be a tomographic image, a composite two-dimensional image, a general schema diagram, and the like. The tomographic image is a reconstructed image generated by reconstructing the series of a plurality of projection images obtained by tomosynthesis imaging. The tomosynthesis imaging is an imaging method in which the mammography apparatus 10 acquires the series of the plurality of projection images by irradiating the breast M with radiation at a plurality of angles. The composite two-dimensional image is a pseudo two-dimensional projection image generated by performing combination processing using a plurality of reconstructed images.

Further, the control unit 40 may comprise a pattern matching processing unit that searches for a region corresponding to the lesion region A detected by the lesion region detection processing from the tomographic image or the composite two-dimensional image. In this case, for example, the control unit 40 outputs a frame or the like indicating the corresponding region detected by the pattern matching processing unit via the search, in a form of being superimposed on the tomographic image or the composite two-dimensional image.

In addition, the control unit 40 may search for the region corresponding to the lesion region A detected by the lesion region detection processing from one projection image, via the pattern matching processing unit. In this case, the control unit 40 specifies a region corresponding to the corresponding region detected by the pattern matching processing unit via the search on the tomographic image, and displays the specified region with a frame or the like.

In addition, in the above-described embodiment and respective modification examples, as a hardware structure of a processing unit that executes various kinds of processing, such as the imaging control unit 50, the image acquisition unit 51, the difference image generation unit 52, the lesion region detection processing unit 53, the detection position comparison determination processing unit 54, and the display control unit 55, various processors shown below can be used.

The various processors include a graphics processing unit (GPU) as well as a CPU. Further, the various processors include, in addition to a general-purpose processor which executes software (program) and functions as various processing units, such as a CPU, a programmable logic device (PLD) that is a processor whose circuit configuration can be changed after manufacture, such as a field programmable gate array (FPGA), and a dedicated electrical circuit that is a processor having a circuit configuration which is designed for exclusive use in order to execute specific processing, such as an application-specific integrated circuit (ASIC).

One processing unit may be configured by one of the various processors or may be configured by combining two or more processors of the same type or different types (for example, by combining a plurality of FPGAs or combining a CPU and an FPGA). Further, a plurality of the processing units may be configured by one processor.

A first example of the configuration in which the plurality of processing units are configured by one processor is a form in which one processor is configured by combining one or more CPUs and the software and this processor functions as the plurality of processing units, as represented by computers such as a client and a server. A second example is a form of using a processor that implements the function of the entire system including the plurality of processing units via one integrated circuit (IC) chip, as represented by a system on a chip (SoC) or the like. In this way, as the hardware structure, the various processing units are configured by using one or more of the various processors described above.

Further, the hardware structure of the various processors is, more specifically, an electric circuit (circuitry) in which circuit elements such as semiconductor elements are combined.

In addition, in the above-described embodiment and respective modification examples, the aspect has been described in which the program 42A is stored in the storage unit 42 in advance, but the present disclosure is not limited to this. The program 42A may be provided in a form of being recorded in a non-transitory recording medium such as a compact disc read only memory (CD-ROM), a digital versatile disc read only memory (DVD-ROM), or a universal serial bus (USB) memory. Further, the program 42A may be downloaded from an external apparatus via a network.

The above-described embodiment and respective modification examples can be combined as appropriate as long as there is no contradiction.

The above-described contents and the above-shown contents are detailed descriptions of portions related to the present disclosed technology and are merely examples of the present disclosed technology. For example, the description of the configuration, the function, the operation, and the effect are the description of examples of the configuration, the function, the operation, and the effect of the parts according to the present disclosed technology. Therefore, it goes without saying that unnecessary parts may be deleted, new elements may be added, or replacements may be made with respect to the above-described contents and the above-shown contents within a range that does not deviate from the gist of the present disclosed technology. Further, the description of, for example, common technical knowledge that does not need to be particularly described to enable the implementation of the present disclosed technology is omitted in the above-described contents and the above-shown contents in order to avoid confusion and to facilitate the understanding of the portions related to the present disclosed technology.

All of the documents, the patent applications, and the technical standards described in the present specification are incorporated into the present specification by reference to the same extent as in a case in which the individual documents, patent applications, and technical standards are specifically and individually stated to be described by reference.

The following technology can be understood from the above description.

## Claims

1. An information processing apparatus comprising:
at least one processor (12),
wherein the processor is configured to:
generate a difference image representing a difference between a low-energy image captured by irradiating a subject, in which a contrast agent is injected, with radiation having first energy and a high-energy image captured by irradiating the subject with radiation having second energy higher than the first energy, by pixel-wise subtracting a weighted version of the low-energy image from a weighted version of the high-energy image;
detect a lesion region including a lesion from each of the low-energy image and the difference image;
perform a comparison determination of a position of the lesion region detected from the low-energy image and a position of the lesion region detected from the difference image, **characterized in that**
for the comparison determination, the processor (12) is configured to:
detect the lesion region from the low-energy image by inputting the low-energy image to a first machine learned model;
detect the lesion region from the difference image by inputting the difference image and, optionally, the low-energy image, to a second machine learned model;
classify each of the lesion regions into a first lesion region detected by both the first machine learned model and the second machine learned model, a second lesion region detected only by the first machine learned model, and a third lesion region detected only by the second machine learned model; and
output a classification result as the comparison determination result.

2. The information processing apparatus according to any preceding claim,
wherein the processor (12) is configured to:
display the comparison determination result on a display (46) along with the low-energy image and the difference image.

3. The information processing apparatus according to any preceding claim,
wherein the processor is configured to:
selectively display the low-energy image and the difference image on a display (46) along with the comparison determination result in response to an operation signal transmitted from an operation unit.

4. The information processing apparatus according to any preceding claim,
wherein the processor is configured to:
display a finding on a display (46) along with the comparison determination result.

5. The information processing apparatus according to any preceding claim,
wherein the subject is left and right breasts,
the low-energy image includes a first low-energy image and a second low-energy image that are captured by irradiating each of the left and right breasts with radiation having the first energy,
the high-energy image includes a first high-energy image and a second high-energy image that are captured by irradiating each of the left and right breasts with radiation having the second energy, and
the difference image includes a first difference image representing a difference between the first low-energy image and the first high-energy image and a second difference image representing a difference between the second low-energy image and the second high-energy image.

6. The information processing apparatus according to claim 5,
wherein the processor is configured to:
selectively display the first low-energy image, the second low-energy image, the first difference image, and the second difference image on a display along with the comparison determination result in response to an operation signal transmitted from an operation unit.

7. An information processing method comprising:
generating a difference image representing a difference between a low-energy image captured by irradiating a subject, in which a contrast agent is injected, with radiation having first energy and a high-energy image captured by irradiating the subject with radiation having second energy higher than the first energy, by pixel-wise subtracting a weighted version of the low-energy image from a weighted version of the high-energy image;
detecting a lesion region including a lesion from each of the low-energy image and the difference image;
performing a comparison determination of a position of the lesion region detected from the low-energy image and a position of the lesion region detected from the difference image,
**characterised in that** the method further comprises:
detecting the lesion region from the low-energy image by inputting the low-energy image to a first machine learned model;
detecting the lesion region from the difference image by inputting the difference image and, optionally, the low-energy image, to a second machine learned model;
classifying each of the lesion regions into a first lesion region detected by both the first machine learned model and the second machine learned model, a second lesion region detected only by the first machine learned model, and a third lesion region detected only by the second machine learned model; and
outputting a classification result as the comparison determination result.

8. A computer program comprising instructions which, when executed by a computer, cause the computer to carry out the method according to claim 7.

## Patentansprüche

1. Informationsverarbeitungsvorrichtung, umfassend:
mindestens einen Prozessor (12),
wobei der Prozessor so konfiguriert ist, dass er:
ein Differenzbild, das eine Differenz zwischen einem Niedrigenergiebild, das durch Bestrahlen eines Untersuchungsobjekts, in das ein Kontrastmittel injiziert worden ist, mit Strahlung, die erste Energie aufweist, aufgenommen wird, und einem Hochenergiebild, das durch Bestrahlen des Untersuchungsobjekts mit Strahlung, die zweite Energie, die höher als die erste Energie ist, aufweist, aufgenommen wird, darstellt, durch pixelweises Subtrahieren einer gewichteten Version des Niedrigenergiebildes von einer gewichteten Version des Hochenergiebildes erzeugt;
einen Läsionsbereich, der eine Läsion enthält, aus jeweils dem Niedrigenergiebild und dem Differenzbild detektiert;
eine Vergleichsbestimmung einer Position des von dem Niedrigenergiebild detektierten Läsionsbereichs und einer Position des von dem Differenzbild detektierten Läsionsbereichs durchführt, **dadurch gekennzeichnet, dass** der Prozessor (12) für die Vergleichsbestimmung so konfiguriert ist, dass er:
den Läsionsbereich aus dem Niedrigenergiebild detektiert, indem er das Niedrigenergiebild in ein erstes maschinell gelerntes Modell eingibt;
den Läsionsbereich aus dem Differenzbild detektiert, indem er das Differenzbild und optional das Niedrigenergiebild in ein zweites maschinell gelerntes Modell eingibt;
jeden der Läsionsbereiche in einen ersten Läsionsbereich, der sowohl von dem ersten maschinell gelernten Modell als auch von dem zweiten maschinell gelernten Modell detektiert worden ist, einen zweiten Läsionsbereich, der nur von dem ersten maschinell gelernten Modell detektiert worden ist, und einen dritten Läsionsbereich, der nur von dem zweiten maschinell gelernten Modell detektiert worden ist, klassifiziert; und
ein Klassifizierungsergebnis als das Vergleichsbestimmungsergebnis ausgibt.

2. Informationsverarbeitungsvorrichtung nach einem der vorhergehenden Ansprüche,
wobei der Prozessor (12) so konfiguriert ist, dass er:
das Vergleichsbestimmungsergebnis auf einer Anzeige (46) zusammen mit dem Niedrigenergiebild und dem Differenzbild anzeigt.

3. Informationsverarbeitungsvorrichtung nach einem der vorhergehenden Ansprüche,
wobei der Prozessor so konfiguriert ist, dass er:
das Niedrigenergiebild und das Differenzbild auf einer Anzeige (46) zusammen mit dem Vergleichsbestimmungsergebnis als Reaktion auf ein von einer Bedienungseinheit übermitteltes Betriebssignal selektiv anzeigt.

4. Informationsverarbeitungsvorrichtung nach einem der vorhergehenden Ansprüche,
wobei der Prozessor so konfiguriert ist, dass er:
einen Befund auf einer Anzeige (46) zusammen mit dem Vergleichsbestimmungsergebnis anzeigt.

5. Informationsverarbeitungsvorrichtung nach einem der vorhergehenden Ansprüche,
wobei das Untersuchungsobjekt eine linke und eine rechte Brust sind,
das Niedrigenergiebild ein erstes Niedrigenergiebild und ein zweites Niedrigenergiebild, die durch Bestrahlen jeweils der linken und rechten Brust mit Strahlung, die die erste Energie aufweist, aufgenommen werden, enthält,
das Hochenergiebild ein erstes Hochenergiebild und ein zweites Hochenergiebild, die durch Bestrahlen jeweils der linken und rechten Brust mit Strahlung, die die zweite Energie aufweist, aufgenommen werden, enthält, und
das Differenzbild ein erstes Differenzbild, das eine Differenz zwischen dem ersten Niedrigenergiebild und dem ersten Hochenergiebild darstellt, und ein zweites Differenzbild, das eine Differenz zwischen dem zweiten Niedrigenergiebild und dem zweiten Hochenergiebild darstellt, enthält.

6. Informationsverarbeitungsvorrichtung nach Anspruch 5,
wobei der Prozessor so konfiguriert ist, dass er:
das erste Niedrigenergiebild, das zweite Niedrigenergiebild, das erste Differenzbild und das zweite Differenzbild auf einer Anzeige zusammen mit dem Vergleichsbestimmungsergebnis als Reaktion auf ein von einer Bedienungseinheit übermitteltes Betriebssignal selektiv anzeigt.

7. Informationsverarbeitungsverfahren, umfassend:
Erzeugen eines Differenzbildes, das eine Differenz zwischen einem Niedrigenergiebild, das durch Bestrahlen eines Untersuchungsobjekts, in das ein Kontrastmittel injiziert worden ist, mit Strahlung, die erste Energie aufweist, aufgenommen wird, und einem Hochenergiebild, das durch Bestrahlen des Untersuchungsobjekts mit Strahlung, die zweite Energie, die höher als die erste Energie ist, aufweist, aufgenommen wird, darstellt, durch pixelweises Subtrahieren einer gewichteten Version des Niedrigenergiebildes von einer gewichteten Version des Hochenergiebildes;
Detektieren eines Läsionsbereichs, der eine Läsion enthält, aus jeweils dem Niedrigenergiebild und dem Differenzbild;
Durchführen einer Vergleichsbestimmung einer Position des von dem Niedrigenergiebild detektierten Läsionsbereichs und einer Position des von dem Differenzbild detektierten Läsionsbereichs, **dadurch gekennzeichnet, dass** das Verfahren ferner umfasst:
Detektieren des Läsionsbereichs aus dem Niedrigenergiebild, indem das Niedrigenergiebild in ein erstes maschinell gelerntes Modell eingegeben wird;
Detektieren des Läsionsbereichs aus dem Differenzbild, indem das Differenzbild und optional das Niedrigenergiebild in ein zweites maschinell gelerntes Modell eingegeben wird;
Klassifizieren jedes der Läsionsbereiche in einen ersten Läsionsbereich, der sowohl von dem ersten maschinell gelernten Modell als auch von dem zweiten maschinell gelernten Modell detektiert worden ist, einen zweiten Läsionsbereich, der nur von dem ersten maschinell gelernten Modell detektiert worden ist, und einen dritten Läsionsbereich, der nur von dem zweiten maschinell gelernten Modell detektiert worden ist; und
Ausgeben eines Klassifizierungsergebnisses als das Vergleichsbestimmungsergebnis.

8. Computerprogramm, das Anweisungen umfasst, die, wenn sie von einem Computer ausgeführt werden, den Computer veranlassen, das Verfahren nach Anspruch 7 durchzuführen.

## Revendications

1. Appareil de traitement d'informations comprenant :
au moins un processeur (12),
dans lequel le processeur est configuré pour :
générer une image de différence représentant une différence entre une image à faible énergie capturée en irradiant un sujet, dans lequel un agent de contraste est injecté, avec un rayonnement ayant une première énergie et une image à haute énergie capturée en irradiant le sujet avec un rayonnement ayant une deuxième énergie supérieure à la première énergie, en soustrayant pixel par pixel une version pondérée de l'image à faible énergie d'une version pondérée de l'image à haute énergie ;
détecter une région de lésion incluant une lésion à partir de chacune de l'image à faible énergie et de l'image de différence ;
effectuer une détermination comparative d'une position de la région de lésion détectée à partir de l'image à faible énergie et d'une position de la région de lésion détectée à partir de l'image de différence, **caractérisé en ce que** pour la détermination comparative, le processeur (12) est configuré pour :
détecter la région de lésion à partir de l'image à faible énergie en entrant l'image à faible énergie dans un premier modèle appris par machine ;
détecter la région de lésion à partir de l'image de différence en entrant l'image de différence et, éventuellement, l'image à faible énergie, dans un deuxième modèle appris par machine ;
classer chacune des régions de lésion en une première région de lésion détectée à la fois par le premier modèle appris par machine et le deuxième modèle appris par machine, une deuxième région de lésion détectée uniquement par le premier modèle appris par machine, et une troisième région de lésion détectée uniquement par le deuxième modèle appris par machine ; et
sortir un résultat de classification en tant que résultat de détermination comparative.

2. Appareil de traitement d'informations selon l'une quelconque des revendications précédentes,
dans lequel le processeur (12) est configuré pour :
afficher le résultat de détermination comparative sur un affichage (46) conjointement avec l'image à faible énergie et l'image de différence.

3. Appareil de traitement d'informations selon l'une quelconque des revendications précédentes,
dans lequel le processeur est configuré pour :
afficher sélectivement l'image à faible énergie et l'image de différence sur un affichage (46) conjointement avec le résultat de détermination comparative en réponse à un signal d'opération transmis par une unité d'opération.

4. Appareil de traitement d'informations selon l'une quelconque des revendications précédentes,
dans lequel le processeur est configuré pour :
afficher une observation médicale sur un affichage (46) conjointement avec le résultat de détermination comparative.

5. Appareil de traitement d'informations selon l'une quelconque des revendications précédentes,
dans lequel le sujet est des seins gauche et droit,
l'image à faible énergie inclut une première image à faible énergie et une deuxième image à faible énergie qui sont capturées en irradiant chacun des seins gauche et droit avec un rayonnement ayant la première énergie,
l'image à haute énergie inclut une première image à haute énergie et une deuxième image à haute énergie qui sont capturées en irradiant chacun des seins gauche et droit avec un rayonnement ayant la deuxième énergie, et
l'image de différence inclut une première image de différence représentant une différence entre la première image à faible énergie et la première image à haute énergie et une deuxième image de différence représentant une différence entre la deuxième image à faible énergie et la deuxième image à haute énergie.

6. Appareil de traitement d'informations selon la revendication 5,
dans lequel le processeur est configuré pour :
afficher sélectivement la première image à faible énergie, la deuxième image à faible énergie, la première image de différence et la deuxième image de différence sur un affichage conjointement avec le résultat de détermination comparative en réponse à un signal d'opération transmis par une unité d'opération.

7. Procédé de traitement d'informations comprenant :
générer une image de différence représentant une différence entre une image à faible énergie capturée en irradiant un sujet, dans lequel un agent de contraste est injecté, avec un rayonnement ayant une première énergie et une image à haute énergie capturée en irradiant le sujet avec un rayonnement ayant une deuxième énergie supérieure à la première énergie, en soustrayant pixel par pixel une version pondérée de l'image à faible énergie d'une version pondérée de l'image à haute énergie ;
détecter une région de lésion incluant une lésion à partir de chacune de l'image à faible énergie et de l'image de différence ;
effectuer une détermination comparative d'une position de la région de lésion détectée à partir de l'image à faible énergie et d'une position de la région de lésion détectée à partir de l'image de différence, **caractérisé en ce que** le procédé comprend en outre :
détecter la région de lésion à partir de l'image à faible énergie en entrant l'image à faible énergie dans un premier modèle appris par machine ;
détecter la région de lésion à partir de l'image de différence en entrant l'image de différence et, éventuellement, l'image à faible énergie, dans un deuxième modèle appris par machine ;
classer chacune des régions de lésion en une première région de lésion détectée à la fois par le premier modèle appris par machine et le deuxième modèle appris par machine, une deuxième région de lésion détectée uniquement par le premier modèle appris par machine, et une troisième région de lésion détectée uniquement par le deuxième modèle appris par machine ; et
sortir un résultat de classification en tant que résultat de détermination comparative.

8. Programme informatique comprenant des instructions qui, lorsqu'elles sont exécutées par un ordinateur, amènent l'ordinateur à effectuer le procédé selon la revendication 7.
